# EUROPEAN PATENT SPECIFICATION

(11) **EP 3 397 980 B1**
(45) Date of publication and mention of the grant of the patent: **21.07.2021**
(21) Application number: 16822220.6
(22) Date of filing: 30.12.2016
(51) Int. Cl.: G01R 33/56, G01R 33/48, A61B 5/00, A61B 5/055, G16H 30/40, G16H 40/63, G16H 40/67, G16H 30/20, A61B 5/1455

(54) **METHOD AND SYSTEM FOR COMBINING DATA FROM A PLURALITY OF MEDICAL DEVICES**
VERFAHREN UND SYSTEM ZUR KOMBINATION VON DATEN VON EINER MEHRZAHL MEDIZINISCHER GERÄTE
PROCÉDÉ ET SYSTÈME DE COMBINAISON DE DONNÉES D'UNE PLURALITÉ D'APPAREILS MÉDICAUX

(30) Priority: 31.12.2015 US 201562274039 P
(43) Date of publication of application: 07.11.2018
(73) Proprietor: Koninklijke Philips N.V., 5656 AG Eindhoven (NL)
(72) Inventor: CRONIN, John, 5656 AE Eindhoven (NL); WILSON, Dylan Jonathan, 5656 AE Eindhoven (NL)
(74) Representative: Philips Intellectual Property & Standards
(86) International application number: PCT/EP2016/082924
(87) International publication number: WO 2017/114951

(56) References cited:
- EP-A1- 1 386 302
- US-A- 5 323 776
- US-A1- 2013 015 975
- US-A1- 2013 211 230
- US-A1- 2013 211 235
- US-A1- 2014 235 975
- FRAUENRATH T. ET AL.: "Improved Cardiac Triggering by Combining Multiple Physiolgocial Signals: A Cardiac MR Feasibility Study at 7.0 T", PROCEEDINGS OF THE INTERNATIONAL SOCIETY FOR MAGNETIC RESONANCE IN MEDICINE, 20TH ANNUAL MEETING, 5 May 2012 (2012-05-05), page 90, XP040622527, Australia

## Description

### BACKGROUND

### Field of the Invention

The present invention generally relates to collecting and merging data from a series of data streams. More specifically, the present invention displays merged data as it is collected for display on a display.

### Description of the Related Art

Magnetic-resonance imagers (MRIS) are used today by doctors collecting images of the body and organs of patients. Currently when a patient is scanned by a magnetic-resonance imaging MRI other medical instruments may sometimes be attached to the patient. In certain instances these instruments provide data that may be recorded for future reference, this data however, is not collected and combined with data collected by an MRI device. Today data collected by an MRI device and other devices in real time are also not merged or fused into data sets that may be referenced and reviewed later in time. Since such fused data may include useful information when viewed as a data set recorded in real time, what is needed are systems and methods that collect, combine (merge/fuse), store, and manipulate fused data sets. US 2013/015975 A1 discloses a method of communicating medical sensing data including receiving, at a patient communication system, medical sensing data collected by a medical sensing device during a medical sensing procedure. The method also includes creating, with the patient communication system, a data message, the data message including at least a portion of the medical sensing data, information about the medical sensing procedure, and information about the medical sensing data and transmitting, with the patient communication system, the data message to a centralized computing device over a data network.

### SUMMARY OF THE PRESENTLY CLAIMED INVENTION

According to a first aspect, the invention concerns a method for collecting and combining data from a plurality of medical devices, as defined in claim 1. A second aspect of the invention concerns a system for collecting and combining data from a plurality of medical devices, as defined in claim 8.

### BRIEF DESCRIPTION OF THE DRAWINGS:

FIG. 1 illustrates a fused data monitor collecting data over time from different pieces of medical equipment or sensors collecting physiological data of a patient when a magnetic-resonance image of the patient is being performed.
FIG. 2 illustrates an exemplary flowchart consistent with a method of the present invention.
FIG. 3 is a block diagram of a device for implementing the present technology.
FIG. 4 illustrates an exemplary method where user input changes medical data displayed on a display.
FIG. 5 illustrates an embodiment for the formation of a fused data stream from data collected over time from different pieces of medical equipment or sensors.
FIG. 6 is a block diagram of an embodiment of a system for implementing the data fusions processes disclosed herein.
FIG. 7 is a flowchart illustrating a method for filtering data.
FIG. 8 is a flowchart illustrating a method for generating a single context data stream.
FIG. 9 is a flowchart illustrating a method for fusing the digital sensor data streams and the context data streams.
FIG. 10 is a flowchart illustrating a method for augmenting or alternatively, defusing a fused digital data stream.
FIG. 11 is a block diagram illustrating a portion of a fused data stream with code bits for defusing the data stream

### DETAILED DESCRIPTION

Embodiments of the present invention generally relate to a system and method for collecting and merging physiological data from a plurality of medical devices and sensors where merged physiological data from the medical devices is sent to a magnetic-resonance imaging (MRI) display device. MRI scan data and data from the plurality of medical devices may be collected in real time and may also be sent over a computer network for storage. Alternatively data may be sent over one or more computer networks and merged at a remote computing device. As such, when data is merged at a remote computing device, time information relating to when data was measured or received may be used to merge data from one or more medical devices and with MRI data maintaining coherency of the data.

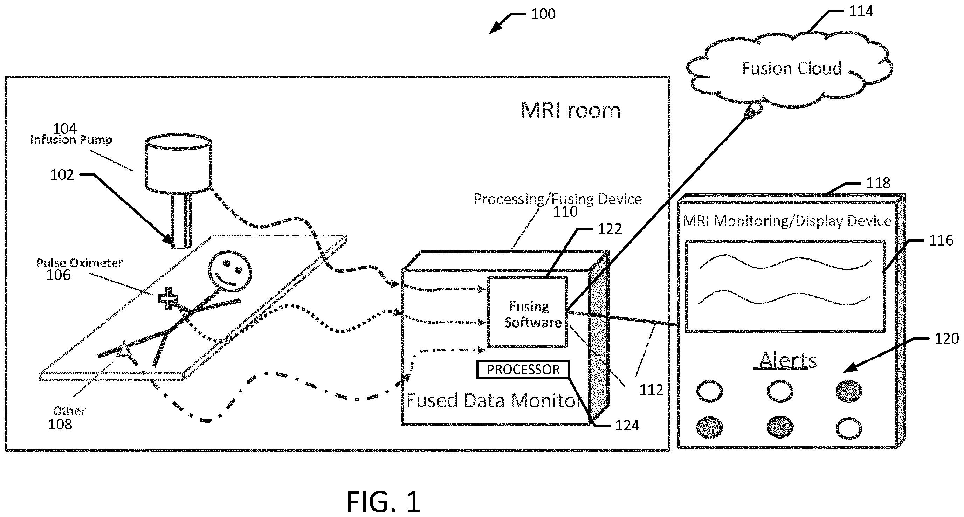

FIG. 1 illustrates a fused data monitor environment 100 collecting data over time from different pieces of medical equipment or sensors collecting physiological data of a patient 102 when a magnetic-resonance image of the patient is being performed. The fused data monitor 110 of FIG. 1 receives data from an infusing pump 104, a pulse oximeter 106 (oxygen monitor), and one other sensor or device. Data collected from the infusing pump 104, the pulse oximeter 106, and the other sensor is fused into a single data stream 112 and is sent to a fused data monitor (i.e. a computing device) 110 that collects and may display magnetic-resonance image (MRI) data of the patient 102. Data collected from these different medical devices may be received over a cable (wire or optical) or may be received wirelessly. Once data is collected it may be merged/fused by any available method. The fusing of data may occur continuously and for as long a desired or required. Data collected and fused by the fused data monitor 110 may also be sent to the cloud 114 or over a computer network for storage. In certain instances the display screen of a commercially available MRI device is augmented to display data collected by external medical devices connected to or monitoring a patient 102. Such augmentation may be implemented using an application program or be incorporated into software source code of the MRI device scanner.

Data displayed on the display 116 at an MRI monitoring device 118 may include MRI data and data from the fused data stream 112. As such, a doctor could see an infusion rate provided by the infusion pump, blood oxygen levels from the pulse oximeter 106, and other sensor data from the other sensor, and MRI data at the same time. This data may be displayed in real time or in near real time. The MRI monitor118 may also provide alerts to doctors or technicians operating the MRI equipment, as indicated by the oval alert indicators of FIG. 1.

The information from the fused data and from the MRI data may be viewed by a clinician or a doctor when identifying the condition of a patient 102. In certain instances, the clinician or doctor may adjust how the data is presented on a display by adjusting settings or by dragging and dropping windows containing data in a user interface at a computing device that has received or that is currently receiving the data. As such, data viewed by a clinician or doctor may be presented differently each time it is "played back" or re-viewed by the clinician or doctor.

In certain instances data collected by a plurality of medical devices may be sent over one or more computer networks and be fused at a computing device 110 on the computer network or at the cloud/Internet 114. In instances where the data is stored remotely or when the data is streamed through a fused data monitor 110 it may be combined with MRI data collected concurrently with the data collected by the medical devices. As such, a doctor or clinician may view all of this combined data on a display either in an MRI data monitor or on a computer anywhere in the world.

Apparatus and methods consistent with the present disclosure provide MRI data to be combined/fused with other collected data giving clinicians an unprecedented ability to monitor interactions in real time. For example, blood oxygen levels collected by a blood oxygen detector and while blood vessels in the body (brain, heart, lung, or other organ) collected by an MRI device may be combined with drug administration data collected by an infusing pump 104 in real time. In an instance where blood vessels were observed to be dilating in the lungs when a drug was administered and when blood oxygen levels were increasing, a doctor may identify that the drug works to provide better oxygen absorption by helping blood vessels in the lungs dilate.

A user interacting with an apparatus and methods consistent with the present disclosure may use a graphical user interface (GUI) displayed on a computing device when configuring and viewing some or all of the fused data 112 in real time, near real time, or in slow motion. The fused data 112 may be stored in data sets stored continuously in real time where at any point in time the fused data represents data collected as a patient 102 was undergoing an MRI scan. The fused data 112 may be analyzed by a processor executing instructions out of memory according to one or more algorithms that measure or identify possible relationships or interactions. Relationships or interactions identified or highlighted by the processor executing instructions according to the algorithms may be sent, presented to, or reviewed by a clinician in real time or at a later time. As such, methods of the present invention include a computing device identifying possible relationships that may be viewed by a clinician only after they have been highlighted by the computing device.

In certain instances, data from two or more patients undergoing the same treatments may be viewed concurrently over time. This may occur even when the data from each respective patient was recorded on different days. In such instances, data collected from patient 1 may be synchronized with patient 2 data and displayed on a display that shows patient 1 data and patient 2 data at the same time. A doctor interacting with a GUI may view this data slowly (slow motion), quickly (fast forward), or frame by frame. Relative rates of time that the data is displayed may also be varied. For example, while data from patient 1 is viewed at a normal (real) time, data collected from patient 2 may be viewed at a multiple speed of 1.3x normal time (or sub-multiple 0.8x normal time). This may allow a doctor to see similar effects in different patients that occur at different rates.

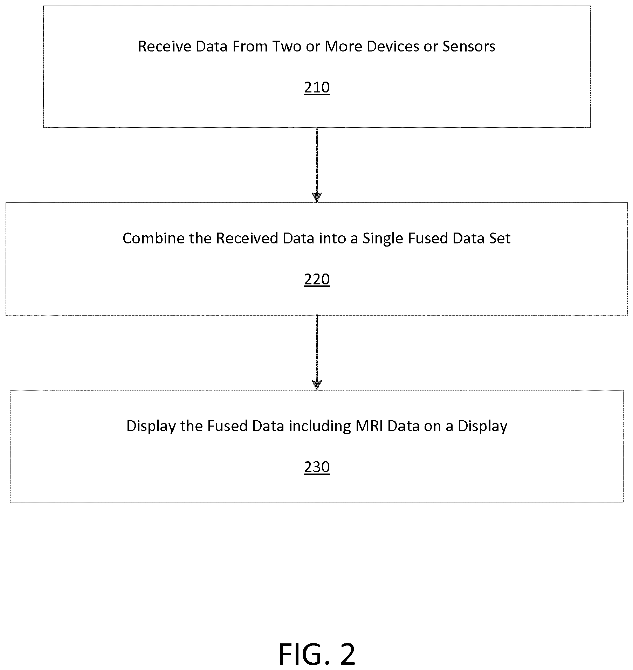

FIG. 2 illustrates an exemplary flowchart consistent with a method of the present invention. FIG. 1 begins with a first step 210 where data is received from two or more medical devices or sensors and in step 220 the received data is combined into a single data set. The single data set may be a stream of data that is stored locally or that is sent over a computer network for storage. Finally in step 230, the combined/fused data 112 is displayed on a display with data collected from an MRI scan of a patient 102.

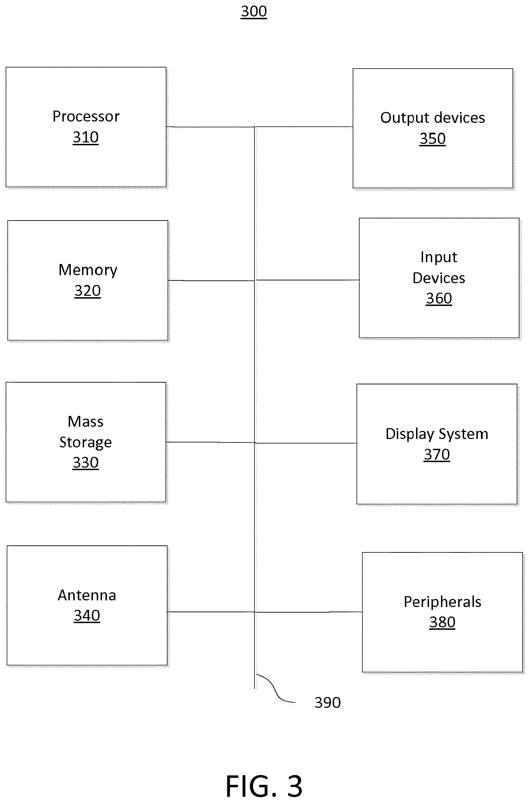

FIG. 3 is a block diagram of a device for implementing the present technology. FIG. 3 illustrates an exemplary computing system 300 that may be used to implement a computing device for use with the present technology. The computing system 300 of FIG. 3 includes one or more processors 310 and memory 320. Main memory 320 may store, in part, instructions and data for execution by processor 310. Main memory can store the executable code when in operation. The system 300 of FIG. 3 further includes a storage 320, which may include mass storage and portable storage, antenna 340, output devices 350, user input devices 360, a display system 370, and peripheral devices 380.

The components shown in FIG. 3 are depicted as being connected via a single bus 390. However, the components may be connected through one or more data transport means. For example, processor unit 310 and main memory 320 may be connected via a local microprocessor bus, and the storage 330, peripheral device(s) 380 and display system 370 may be connected via one or more input/output (I/O) buses.

Storage device 330, which may include mass storage implemented with a magnetic disk drive or an optical disk drive, may be a non-volatile storage device for storing data and instructions for use by processor unit 310. Storage device 330 can store the system software for implementing embodiments of the present invention for purposes of loading that software into main memory 310.

Portable storage device of storage 330 operates in conjunction with a portable non-volatile storage medium, such as a floppy disk, compact disk or Digital video disc, to input and output data and code to and from the computer system 300 of FIG. 3. The system software for implementing embodiments of the present invention may be stored on such a portable medium and input to the computer system 300 via the portable storage device.

Antenna 340 may include one or more antennas for communicating wirelessly with another device. Antenna 340 may be used, for example, to communicate wirelessly via Wi-Fi, Bluetooth, with a cellular network, or with other wireless protocols and systems. The one or more antennas may be controlled by a processor 310, which may include a controller, to transmit and receive wireless signals. For example, processor 310 execute programs stored in memory 320 to control antenna 340 transmit a wireless signal to a cellular network and receive a wireless signal from a cellular network.

The system 300 as shown in FIG. 3 includes output devices 350 and input device 360. Examples of suitable output devices include speakers, printers, network interfaces, and monitors. Input devices 360 may include a touch screen, microphone, accelerometers, a camera, and other device. Input devices 360 may include an alpha-numeric keypad, such as a keyboard, for inputting alpha-numeric and other information, or a pointing device, such as a mouse, a trackball, stylus, or cursor direction keys.

Display system 370 may include a liquid crystal display (LCD), LED display, or other suitable display device. Display system 370 receives textual and graphical information, and processes the information for output to the display device.

Peripherals 380 may include any type of computer support device to add additional functionality to the computer system. For example, peripheral device(s) 380 may include a modem or a router.

The components contained in the computer system 300 of FIG. 3 are those typically found in computing system, such as but not limited to a desk top computer, lap top computer, notebook computer, net book computer, tablet computer, smart phone, personal data assistant (PDA), or other computer that may be suitable for use with embodiments of the present invention and are intended to represent a broad category of such computer components that are well known in the art. Thus, the computer system 300 of FIG. 3 can be a personal computer, hand held computing device, telephone, mobile computing device, workstation, server, minicomputer, mainframe computer, or any other computing device. The computer can also include different bus configurations, networked platforms, multi-processor platforms, etc. Various operating systems can be used including Unix, Linux, Windows, Macintosh OS, Palm OS, and other suitable operating systems.

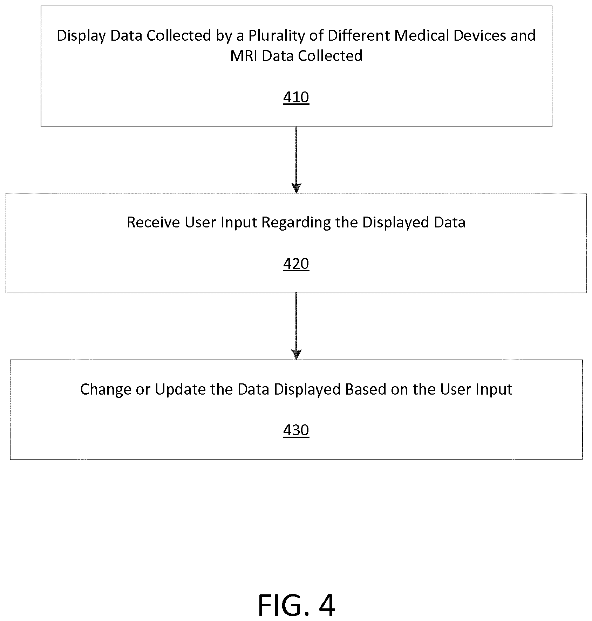

FIG. 4 illustrates an exemplary method where user input changes medical data displayed on a display. Step 410 of FIG. 4 is a step where data collected by a plurality of different medical devices or sensors is displayed on a display. Next step 420 receives user input regarding the data displayed on the display. This user input may be received over a graphical user interface (GUI). The user input may change locations where certain data is displayed, change what data is displayed on the display, or change a playback speed. For example, a user may drag and drop a box that includes pulse rate data to an area of the display preferred by the user. Finally, in step 430 of FIG. 4 the display of data is updated based on the input received from the user.

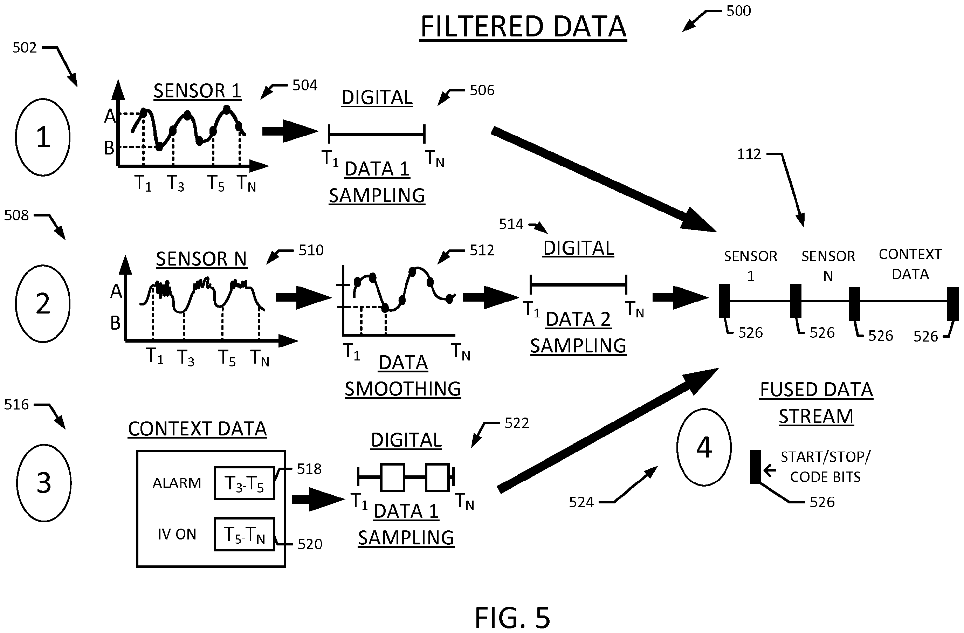

FIG. 5 illustrates an exemplary method 500 for forming a fused data stream 112 from data collected over time from different pieces of medical equipment or sensors. As shown, various sensors, such as sensors 1 thru sensors N may each have their own unique filtering process as generally indicated by 502 and 508. In a first example sensor filtering process 502, the analog signal 504 of Sensor 1 is time sampled to provide digital data 506 from time 1 (T₁) thru Time N (T_{N}). In a second example sensor filtering process 508, Sensor N is time sampled 510 and filtered for noise 512 (i.e. removal of high frequencies) before digital sampling of the signal to generate digital data 514.

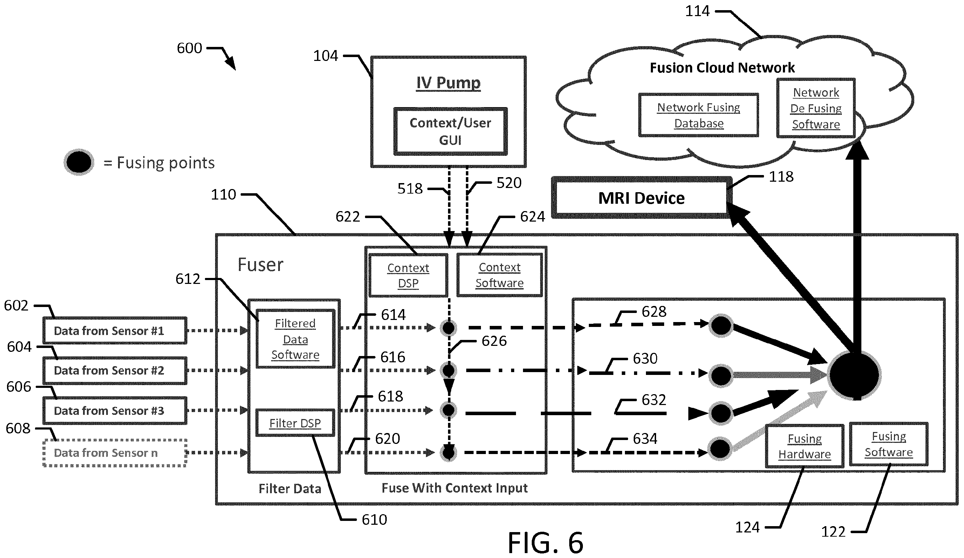

In the same time frame as the filtering indicated by 502 and 508, additional context data is collected in a third example process 516. The context data such as alarms 518 that were triggered between time 3 (T₃) and time 5 (T₅) and IV pump operation data 520 from time 5 (T₅) through time N (T_{N}) is recorded. This data is converted to digital data 522. Lastly the data from each example process 502, 508, and 516 is "fused" in a fourth example process 524. As used herein, fusing the data means processing the data through a fusing system, such as the fused data monitor 110 to join the data streams together into a single data stream. In one aspect, fusing software 122, executing on a processor 124 of the computing device 110, codes each stream with start/stop/code bits 526 that aid in determining which data stream is which during subsequent decoding. In one embodiment, the fused data 112 is sent and stored on a fusion cloud network 114 as well as sent to the MRI device 118, as shown in FIG. 6 which may include its own defusing hardware and software to extract the individual streams from the single fused data stream 112.

FIG. 6 is a block diagram of an embodiment of a system for implementing the data fusions processes disclosed herein. As shown, data 602-608 from sensors 1 thru sensors N is transmitted to the fused data monitor 110, a computing device, which includes a filter digital signal processor (DSP) 610. In one aspect, the filter DSP is a fast digital signal processor that executes instructions found in the filtered data software 612 to filter the data 602-608 in each sensor data stream. The filter DSP 610 also converts each sensor data feeds into individual sensor digital data streams 614-620. As shown, context data 518-520 (e.g. alarms, IV pump on/off etc. as shown in FIG. 5) are received at the fused data monitor 110 from the IV pump 104. A context DSP 622 and context software 624 process and convert the context data 518-520 data into a single context digital data stream 626. The context digital data stream 626 is then fused with one or more of the individual sensor digital data streams 614-620. The one or more combined sensor data and context data streams 628-634 are then feed to a fusing processor 124 that executes fusing software 122. The fusing processor 124 receives the combined sensor data and context data streams 628-634, or alternatively, the individual sensor digital data streams 614-620 and single context digital data stream 626 and creates one digital data stream 112 with start/stop/code bit groups 526 to identify which portions of the fused data stream originate from each sensor and the context data. In one aspect, the fusing software 122 may unpack this data for display at the MRI display device 116, save the data streams to the fusion cloud network 114, or both.

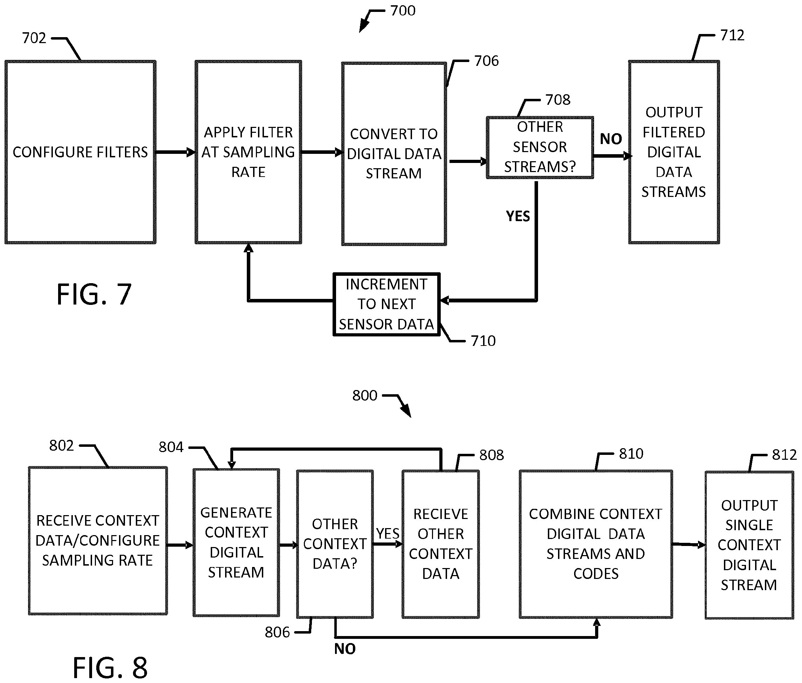

Fig. 7 is a flowchart illustrating a method 700 for filtering data. In one aspect, instructions for performing the method are embodied in data filter software executable by processor. In one aspect, a filtering process is performed on each sensor data stream. In one embodiment, the disclosed filter software directs the filter DSP to filter the sensor data simultaneously. In another embodiment, the disclosed filter software directs the filter DSP to filter data from the first sensor, generate an output for the first sensor, and then progress incrementally through each sensor until all the sensor streams are processed.

In one embodiment, the method 700 includes configuring the filters for each data stream at step 702. By way of example, configuring the filters may include the type of filtering to be performed, the duration of the time sampling, and the overall sampling rate for the data streams. Other filtering processes may be performed. In another example, the filter may be configured such that data from sensor 1 is time sampled, while data from sensor N is smoothed and then time sampled. Regarding the overall sampling rate, it is understood that real time changes for each sensor may occur on the order of seconds. For example, a pulse oximeter does not change its data until one second as elapsed. As such, the sampling rate for the entire process, according to one embodiment, will be a one second cycle. Thus, each sensor will be sampled for a duration equal to the process cycle divided by the number of sensors. In a system with five sensors, each sensor is samples for 1/5th of a second and the sampling process is repeated.

At step 704, data from sensor 1 is received at the filter DSP and the filtering process is applied at the sampling rate determined at step 702. At step 706, the filtered data stream is converted to a digital data stream. A determination is made regarding the receipt of additional sensor data streams at step 708. If additional sensor data streams are received, the method increments to the next sensor data at step 710 and then returns to 704 and filters the next sensor data. If there are no additional data streams identified at 708, the filtered sensor digital streams are output at step 712.

Fig. 8 is a flowchart illustrating a method 800 for generating a single context data stream. In one aspect, instructions for performing the method are embodied in context software executable by processor. In one aspect, a context data processing and sampling process is performed on each context data stream. In one embodiment, the disclosed context software directs a context DSP to process and sample a first context data stream, then progress incrementally through other context streams until all the context data streams are processed.

In one embodiment, the method 800 includes receiving a first context data stream at the context DSP at step 802. By way of example, a context data stream may include alarm data, IV Pump on/off operations, or messages from a machine or computing device. The context data stream may also include time stamps. At step 802, the context DSP also prepares the start/stop code bits 526 and determines the overall sampling rate for running the process 800. Regarding the overall sampling rate, it is understood that real time changes for each machine or peripheral device may occur on the order of seconds. For example, an alarm state does not change its data until one second as elapsed. As such, the sampling rate for the entire process, according to one embodiment, will be a one second cycle. Thus, each context data stream in will be sampled for a duration equal to the process cycle divided by the number of context data streams. In a system with five context data streams, each stream is samples for 1/5th of a second and the sampling process is repeated.

At step 804, the context data is sampled and converted to a context digital data stream, while at step 806, a determination is made regarding the availability of additional context data streams. If additional context data streams are available, the next stream is received at step 808, and the method 800 returns to step 804 where the new context data stream is sampled and converted to a context digital data stream. If there are no additional data streams identified at step 806, the context digital data streams, along with the start/stop identification codes are combined into a single context digital data stream at step 810, which is then output at step 812.

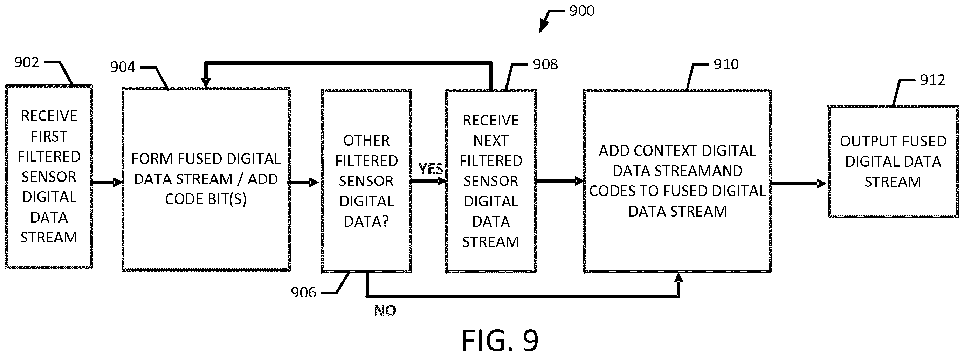

Fig. 9 is a flowchart illustrating a method 900 for fusing the digital sensor data streams and the context data streams. In one aspect, instructions for performing the method are embodied in fusing software executable by processor. In one aspect, a fusing process is performed on each filtered sensor digital data stream. In one embodiment, the disclosed fusing software directs a fusing processor to first combine the filtered sensor digital data streams and then combine the context digital data stream.

In one embodiment, the method 900 includes receiving at the fusing processor at step 902, the first filtered sensor digital data output from the filtering software at step 712, as shown in FIG. 7. At step 904, a single fused digital data stream is formed and start/stop code bits, along with other code bits are added to the stream fused digital data stream. By way of example, the other code bits may include a "code 018" bit to identify the individual sensor 1 filtered digital data stream, while "code 019" identifies the sensor n filtered digital data. At step 906, a determination is made regarding the availability of additional filtered sensor digital data streams. If additional filtered sensor digital data streams are available, the next stream is received at step 908, and the method 900 returns to step 904 where the next filtered sensor digital data stream is added to the fused digital data stream. If there are no additional data streams identified at step 906, then the single context digital data stream output at step 812 by the context software, as shown in FIG. 8, is added to the fused digital data stream at step 910. Additional code bits may be added to the fused digital stream at this point. For example, a "code 021" bit which indicates the start of the context data stream may be added to the fused data stream. This coding process is continued for the next block of filtered sensor data (e.g. 1 second) and block of context data (e.g. 1 second) for a total block of 2 seconds of data. Lastly, at step 912 the fused digital data stream is output and may be transmitted to the network fusing cloud or a database therein and to the MRI device.

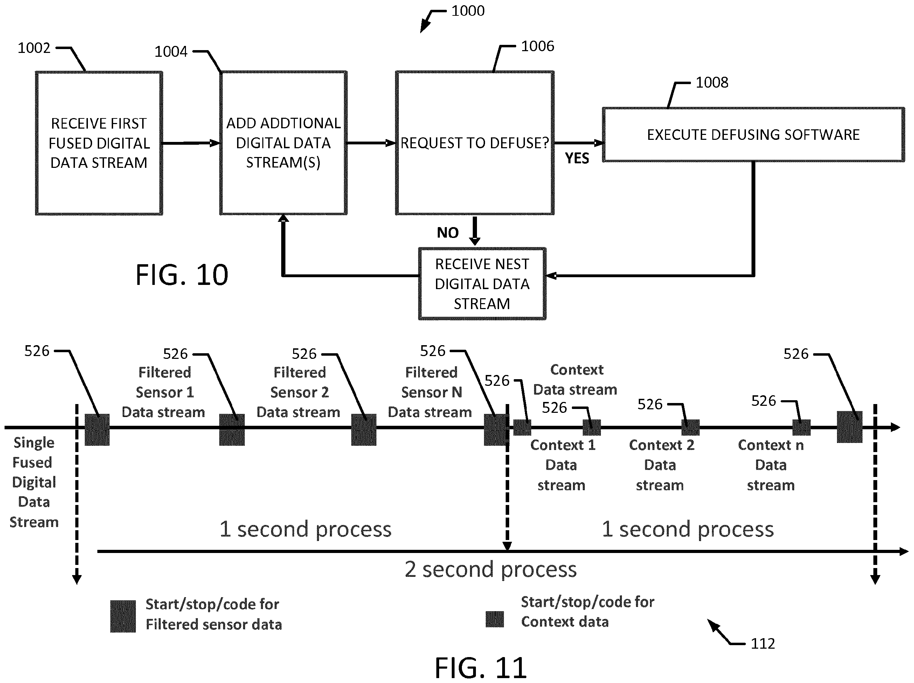

FIGS. 10-11 illustrate a method 1000 for augmenting a fused data stream and defusing a fused digital data stream, such as that generated by the fusing software as shown in FIG. 9. In one aspect, the method may be performed by defusing software executing on a processor. In one embodiment, the method 1000 takes the two seconds worth of the fused digital data stream and stores it for later analysis and defusing. In one aspect, the method 100 defuses the fused data stream using the start/stop code and other code bits and stores those individual data streams and code bits to the memory of the MRI device for subsequent on MRI device display. The process may repeat in two second blocks thus providing a continual flow of real time data. As shown, the method 1000 includes receiving a first digital data stream at step 1002, while at step 1004, additional digital data streams are fused. At step 1006, the method 1000 determines if a request to defuse the data has been received. If a request to defuse is received, then the defusing software is executed at step 1008 to parse the fused digital data stream using the code bits 526 shown in FIG. 11. In one aspect the defusing software may be executed over a network and the defused data may be transmitted to other computing devices in response to requests or to 3rd-party research groups, hospital medical records, doctor, or other personnel. However, if a request to defuse the stream is not received at step 1006, the method may continue to augment the digital data streams at step 1004.

While various embodiments have been described above, it should be understood that they have been presented by way of example only, and not limitation. The descriptions are not intended to limit the scope of the invention to the particular forms set forth herein. Thus, the breadth and scope of a preferred embodiment should not be limited by any of the above-described exemplary embodiments. It should be understood that the above description is illustrative and not restrictive. To the contrary, the present descriptions are intended to cover such alternatives, modifications, and equivalents as covered by the scope of the invention as defined by the appended claims.

The scope of the invention should, therefore, be determined not with reference to the above description, but instead should be determined with reference to the appended claims.

## Claims

1. A method for collecting and combining data from a plurality of medical devices, the method comprising:
receiving, by a processor (310), sensor data from a sensor of a first medical device of the plurality of medical devices;
filtering, by the processor, the sensor data over a period of time; converting, by the processor, the sensor data to digital sensor data (506, 514);
receiving, by the processor, context data (518-520) from a second medical device of the plurality of medical devices over said period of time;
generating identification codes, by the processor, to identify the context data;
generating start/stop code bits, by the processor, to identify a state of the second medical device;
converting, by the processor, the context data into digital context data, the digital context data further comprising the identification codes and the start/stop code bits;
generating sensor identification codes, by a processor, to identify the digital sensor data;
combining (900), by the processor, the digital sensor data and the digital context data into a fused data stream (112), the fused data stream further comprising the sensor identification codes; and,
storing the fused data stream in a database.

2. The method of claim 1, further comprising determining a sampling rate for the sensor data, by the processor (310), wherein said filtering of the sensor data is applied at the determined sampling rate.

3. The method of claim 2, wherein the sampling rate is determined by the ratio of a real time change in sensor data to a number of sensor data streams.

4. The method of claim 1, further comprising smoothing, by the processor (310), the filtered the sensor data.

5. The method of claim 1, further comprising:
receiving, by the processor (310), second sensor data from a sensor of a third medical device of the plurality of medical devices;
filtering, by the processor, the second sensor data over a period of time; and
converting, by the processor, the second sensor data to a second digital sensor data;
generating second sensor identification codes, by the processor, to identify the second digital sensor data; and
combining (900), by the processor, the digital sensor data, the second digital sensor data, and the digital context data into a fused data stream (112), the fused data stream further comprising the sensor identification codes and the second sensor identification codes.

6. The method of claim 1, further comprising:
receiving, by the processor (310), second context data from a fourth medical device of the plurality of medical devices;
generating identification codes, by the processor, to identify the second context data;
generating second start/stop code bits by the processor, to identify a state of the fourth medical device;
converting, by the processor, the second context data into second digital context data, the second digital context data further comprising the second identification codes and the second start/stop code bits; and
combining, by the processor, the digital context data and the second digital context data into a single context data stream.

7. The method of claim 1, wherein at least one of the one or more devices is an IV infusion pump (104) or a pulse oximeter (106).

8. A system (300) for collecting and combining data from a plurality of medical devices, the system comprising:
a memory (320);
a sensor in communication with a first medical device of the plurality of medical devices; and
at least one processor (310) configured to:
receive sensor data from the sensor of the first medical device of the plurality of medical devices;
filter the sensor data over a period of time;
convert the sensor data to digital sensor data;
receive context data (518-520) from a second medical device of the plurality of medical devices over said period of time;
generate identification codes to identify the context data;
generate start/stop code bits to identify a state of the second medical device;
convert the context data in to digital context data, the digital context data further comprising the identification codes and the start/stop code bits;
generate sensor identification codes to identify the digital sensor data;
combine (900) the digital sensor data (506, 514) and the digital context data into a fused data stream, the fused data stream further comprising the sensor identification codes; and,
store the fused data stream in the memory.

9. The system of claim 8, wherein the processor (310) is configured to determine a sampling rate for the sensor data, wherein said filtering of the sensor data is applied at the determined sampling rate.

10. The system of claim 9, wherein the sampling rate is a ratio of a real time change in sensor data to a number of sensor data streams.

11. The system of claim 8, wherein the processor (310) is configured to smooth the filtered sensor data.

12. The system of claim 8, wherein the processor (310) is configured to:
receive second sensor data from a sensor of a third medical device of the plurality of medical devices;
filter the second sensor data over a period of time;
convert the second sensor data to a second digital sensor data;
generate second sensor identification codes to identify the second digital sensor data; and
combine (900) the digital sensor data, the second digital sensor data, and the digital context data into a fused data stream, the fused data stream further comprising the sensor identification codes and the second sensor identification codes.

13. The system of claim 8, wherein the processor (310) is configured to:
receive second context data from a fourth medical device of the plurality of medical devices;
generate identification codes to identify the second context data;
generate second start/stop code bits to identify a state of the fourth medical device;
convert the second context data into second digital context data, the second digital context data further comprising the second identification codes and the second start/stop code bits; and
combine the digital context data and the second digital context data into a single context data stream.

## Patentansprüche

1. Eine Methode zum Erfassen und Kombinieren von Daten mehrerer medizinische Geräte, wobei die Methode folgende Schritte umfasst:
Abrufen der Sensordaten eines Sensors eines ersten medizinischen Geräts der mehreren medizinischen Geräte mit einem Prozessor (310);
Filtern der Sensordaten über einen Zeitraum mit dem Prozessor;
Konvertieren der Sensordaten in digitale Sensordaten (506, 514) mit dem Prozessor;
Abrufen von Kontextdaten (518-520) von einem zweiten medizinischen Gerät der mehreren medizinischen Geräte über den Zeitraum mit dem Prozessor;
Generieren von Identifikationscodes mit dem Prozessor, um die Kontextdaten zu identifizieren;
Generieren von Start/Stop-Codebits mit dem Prozessor, um einen Zustand des zweiten medizinischen Geräts zu ermitteln;
Konvertieren der Kontextdaten in digitale Kontextdaten mit dem Prozessor, wobei die digitalen Kontextdaten zudem die Identifikationscodes und die Start/Stop-Codebits umfassen;
Generieren von Sensor-Identifikationscodes mit dem Prozessor, um die digitalen Sensordaten zu identifizieren;
Kombinieren (900) der digitalen Sensordaten und der digitalen Kontextdaten in einem zusammengeführten Datenstrom (112) mit dem Prozessor, wobei der zusammengeführte Datenstrom zudem die Sensor-Identifikationscodes umfasst; und
Speichern des zusammengeführten Datenstroms in einer Datenbank.

2. Die Methode gemäß Anspruch 1, die zudem das Ermitteln einer Abtastrate für die Sensordaten durch den Prozessor (310) umfasst, wobei das Filtern der Sensordaten mit der ermittelten Abtastrate erfolgt.

3. Die Methode gemäß Anspruch 2, wobei die Abtastrate anhand des Verhältnisses der Echtzeit-Veränderung der Sensordaten zu einer Anzahl von Sensordatenströmen ermittelt wird.

4. Die Methode gemäß Anspruch 1, die zudem das Glätten der gefilterten Sensordaten durch den Prozessor (310) umfasst.

5. Die Methode gemäß Anspruch 1, die zudem fol-gende Schritte umfasst:
Abrufen von zweiten Sensordaten eines Sensors eines dritten medizinischen Geräts der mehreren medizinischen Geräte mit dem Prozessor (310);
Filtern der zweiten Sensordaten über einen Zeitraum mit dem Prozessor; und
Konvertieren der zweiten Sensordaten in zweite digitale Sensordaten mit dem Prozessor;
Generieren von zweiten Sensor-Identifikationscodes mit dem Prozessor, um die zweiten digitalen Sensordaten zu identifizieren; und
Kombinieren (900) der digitalen Sensordaten, der zweiten digitalen Sensordaten und der digitalen Kontextdaten in einem zusammengeführten Datenstrom (112) mit dem Prozessor, wobei der zusammengeführte Datenstrom zudem die Sensor-Identifikationscodes und die zweiten Sensor-Identifikationscodes um-fasst.

6. Die Methode gemäß Anspruch 1, die zudem folgende Schritte umfasst:
Abrufen von zweiten Kontextdaten von einem vierten medizinischen Gerät der mehreren medizinischen Geräte mit dem Prozessor (310);
Generieren von Identifikationscodes mit dem Prozessor, um die zweiten Kontextdaten zu identifizieren;
Generieren von zweiten Start/Stop-Codebits mit dem Prozessor, um einen Zustand des vierten medizinischen Geräts zu ermitteln;
Konvertieren der zweiten Kontextdaten in zweite digitale Kontextdaten mit dem Prozessor, wobei die zweiten digitalen Kontextdaten zudem die zweiten Identifikationscodes und die zweiten Start/Stop-Codebits umfassen; und
Kombinieren der digitalen Kontextdaten und der zweiten digitalen Kontextdaten mit dem Prozessor zu einem einzigen Kontextdatenstrom.

7. Die Methode gemäß Anspruch 1, wobei es sich bei mindestens einem der Geräte um eine IV-Infusionspumpe (104) oder um ein Pulsoximeter (106) handelt.

8. Eine System (300) zum Erfassen und Kombinieren von Daten mehrerer medizinische Geräte, wobei das System Folgendes umfasst:
einen Speicher (320);
einen Sensor, der mit einem ersten medizinischen Gerät der mehreren medizinischen Geräte verbunden ist; und
mindestens einen Prozessor (310), der folgende Schritte durchführt:
Abrufen der Sensordaten vom Sensor eines ersten medizinischen Geräts der mehreren medizinischen Geräte;
Filtern der Sensordaten über einen Zeitraum;
Konvertieren der Sensordaten in digitale Sensordaten;
Abrufen von Kontextdaten (518-520) von einem zweiten medizinischen Gerät der mehreren medizinischen Geräte über den Zeitraum;
Generieren von Identifikationscodes, um die Kontextdaten zu identifizieren;
Generieren von Start/Stop-Codebits, um einen Zustand des zweiten medizinischen Geräts zu ermitteln;
Konvertieren der Kontextdaten in digitale Kontextdaten, wobei die digitalen Kontextdaten zudem die Identifikationscodes und die Start/Stop-Codebits umfassen;
Generieren von Sensor-Identifikationscodes, um die digitalen Sensordaten zu identifizieren;
Kombinieren (900) der digitalen Sensordaten (506, 514) und der digitalen Kontextdaten in einem zusammengeführten Datenstrom, wobei der zusammengeführte Datenstrom zudem die Sensor-Identifikationscodes umfasst; und
Speichern des zusammengeführten Datenstroms auf dem Speicher.

9. Das System gemäß Anspruch 8, wobei der Prozessor (310) eine Abtastrate für die Sensordaten ermittelt, wobei das Filtern der Sensordaten mit der ermittelten Abtastrate erfolgt.

10. Das System gemäß Anspruch 9, wobei es sich bei der Abtastrate um ein Verhältnis der Echtzeit-Veränderung der Sensordaten zu einer Anzahl von Sensordatenströmen handelt.

11. Das System gemäß Anspruch 8, wobei der Prozessor (310) die gefilterten Sensordaten glättet.

12. Das System gemäß Anspruch 8, wobei der Prozessor (310) folgende Schritte durchführt:
Abrufen von zweiten Sensordaten eines Sensors eines dritten medizinischen Geräts der mehreren medizinischen Geräte;
Filtern der zweiten Sensordaten über einen Zeitraum;
Konvertieren der zweiten Sensordaten in zweite digitale Sensordaten;
Generieren von zweiten Sensor-Identifikationscodes, um die zweiten digitalen Sensordaten zu identifizieren; und
Kombinieren (900) der digitalen Sensordaten, der zweiten digitalen Sensordaten und der digitalen Kontextdaten in einem zusammengeführten Datenstrom, wobei der zusammengeführte Datenstrom zudem die Sensor-Identifikationscodes und die zweiten Sensor-Identifikationscodes umfasst.

13. Das System gemäß Anspruch 8, wobei der Prozessor (310) folgende Schritte durchführt:
Abrufen von zweiten Kontextdaten von einem vierten medizinischen Gerät der mehreren medizinischen Geräte;
Generieren von Identifikationscodes, um die zweiten Kontextdaten zu identifizieren;
Generieren von zweiten Start/Stop-Codebits, um einen Zustand des vierten medizinischen Geräts zu ermitteln;
Konvertieren der zweiten Kontextdaten in zweite digitale Kontextdaten, wobei die zweiten digitalen Kontextdaten zudem die zweiten Identifikationscodes und die zweiten Start/Stop-Codebits umfassen; und
Kombinieren der digitalen Kontextdaten und der zweiten digitalen Kontextdaten zu einem einzigen Kontextdatenstrom.

## Revendications

1. Procédé de collecte et de combinaison des données à partir d'une pluralité de dispositifs médicaux, ledit procédé comprenant :
la réception, par un processeur (310), des données de capteur d'un capteur d'un premier dispositif médical de la pluralité de dispositifs médicaux ;
le filtrage, par le processeur, des données de capteur pendant une période de temps ;
la conversion, par le processeur, des données de capteur en données numériques de capteur (506, 514) ;
la réception, par le processeur, des données de contexte (518-520) d'un deuxième dispositif médical de la pluralité de dispositifs médicaux pendant ladite période de temps ;
la génération des codes d'identification, par le processeur, pour identifier les données de contexte ;
la génération des bits de code de démarrage/d'arrêt, par le processeur, pour identifier un état du deuxième dispositif médical ;
la conversion, par le processeur, des données de contexte en données numériques de contextes, les données numériques de contextes comprenant en outre les codes d'identification et les bits de code de démarrage/d'arrêt,
la génération des codes d'identification de capteur, par le processeur, pour identifier les données numériques de capteur ;
la combinaison (900), par le processeur, des données numériques de capteur et des données numériques de contexte en un flux de données fusionnées (112), le flux de données fusionnées comprenant en outre les codes d'identification de capteur ; et,
la mémorisation du flux de données fusionnées dans une base de données.

2. Procédé selon la revendication 1, comprenant en outre la détermination d'une fréquence d'échantillonnage pour les données de capteur, par le processeur (310), dans lequel ledit filtrage des données de capteur est appliqué à la fréquence d'échantillonnage déterminée.

3. Procédé selon la revendication 2, dans lequel la fréquence d'échantillonnage est déterminée par le rapport d'un changement en temps réel des données de capteur sur un certain nombre de flux de données de capteur.

4. Procédé selon la revendication 1, comprenant en outre le lissage, par le processeur (310), des données de capteur filtrées.

5. Procédé selon la revendication 1, comprenant en outre :
la réception, par le processeur (310), des secondes données de capteur provenant d'un capteur d'un troisième dispositif médical de la pluralité de dispositifs médicaux ;
le filtrage, par le processeur, des secondes données de capteur pendant une période de temps ; et
la conversion, par le processeur, des secondes données de capteur en des secondes données numériques de capteur ;
la génération des seconds codes d'identification de capteur, par le processeur, pour identifier les secondes données numériques de capteur ; et
la combinaison (900), par le processeur, des données numériques de capteur, des secondes données numériques de capteur et des données numériques de contexte en un flux de données fusionnées (112), le flux de données fusionnées comprenant en outre les codes d'identification de capteur et les seconds codes d'identification de capteur.

6. Procédé selon la revendication 1, comprenant en outre :
la réception, par le processeur (310), des secondes données de contexte d'un quatrième dispositif médical de la pluralité de dispositifs médicaux ;
la génération des codes d'identification, par le processeur, pour identifier les secondes données de contexte ;
la génération des seconds bits de code de démarrage/d'arrêt, par le processeur, pour identifier un état du quatrième dispositif médical ;
la conversion, par le processeur, des secondes données de contexte en secondes données numériques de contexte, les secondes données numériques de contexte comprenant en outre les seconds codes d'identification et les seconds bits de code de démarrage/d'arrêt ; et
la combinaison, par le processeur, des données numériques de contexte et des secondes données numériques de contexte en un seul flux de données de contexte.

7. Procédé selon la revendication 1, dans lequel l'au moins un des au moins un dispositif est une pompe à perfusion IV (104) ou un oxymètre de pouls (106).

8. Système (300) de collecte et de combinaison des données à partir d'une pluralité de dispositifs médicaux, ledit système comprenant :
une mémoire (320) ;
un capteur en communication avec un premier dispositif médical de la pluralité de dispositifs médicaux ; et
au moins un processeur (310) configuré :
pour recevoir des données de capteur du capteur du premier dispositif médical de la pluralité de dispositifs médicaux ;
pour filtrer les données du capteur pendant une période de temps ;
pour convertir les données de capteur en données numériques de capteur ;
pour recevoir des données de contexte (518-520) d'un deuxième dispositif médical de la pluralité de dispositifs médicaux pendant ladite période de temps ;
pour générer des codes d'identification pour identifier les données de contexte ;
pour générer des bits de code de démarrage/d'arrêt pour identifier un état du deuxième dispositif médical ;
pour convertir les données de contexte en données numériques de contexte, les données numériques de contexte comprenant en outre les codes d'identification et les bits de code de démarrage/d'arrêt ;
pour générer des codes d'identification de capteur pour identifier les données numériques de capteur ;
pour combiner (900) les données numériques de capteur (506, 514) et les données numériques de contexte en un flux de données fusionnées, le flux de données fusionnées comprenant en outre les codes d'identification de capteur ; et,
pour mémoriser le flux de données fusionnées dans la mémoire.

9. Système selon la revendication 8, dans lequel le processeur (310) est configuré
pour déterminer une fréquence d'échantillonnage pour les données de capteur, dans lequel ledit filtrage des données de capteur est appliqué à la fréquence d'échantillonnage déterminée.

10. Système selon la revendication 9, dans lequel la fréquence d'échantillonnage est un rapport d'un changement en temps réel des données de capteur sur un certain nombre de flux de données de capteur.

11. Système selon la revendication 8, dans lequel le processeur (310) est configuré pour lisser les données de capteur filtrées.

12. Système selon la revendication 8, dans lequel le processeur (310) est configuré :
pour recevoir des secondes données de capteur d'un capteur d'un troisième dispositif médical de la pluralité de dispositifs médicaux ;
pour filtrer les secondes données de capteur pendant une période de temps ;
pour convertir les secondes données de capteur en des secondes données numériques de capteur ;
pour générer des seconds codes d'identification de capteur pour identifier les secondes données numériques de capteur ; et
pour combiner (900) les données numériques de capteur, les secondes données numériques de capteur et
les données numériques de contexte en un flux de données fusionnées, le flux de données fusionnées comprenant en outre les codes d'identification de capteur et les seconds codes d'identification de capteur.

13. Système selon la revendication 8, dans lequel le processeur (310) est configuré :
pour recevoir des secondes données de contexte d'un quatrième dispositif médical de la pluralité de dispositifs médicaux ;
pour générer des codes d'identification pour identifier les secondes données de contexte ;
pour générer des seconds bits de code de démarrage/d'arrêt pour identifier un état du quatrième dispositif médical ;
pour convertir les secondes données de contexte en secondes données numériques de contexte, les secondes données numériques de contexte comprenant en outre les seconds codes d'identification et les seconds bits de code de démarrage/d'arrêt ; et
pour combiner les données numériques de contexte et les secondes données numériques de contexte en un seul flux de données de contexte.
